# EUROPEAN PATENT SPECIFICATION

(11) **EP 2 703 529 B1**
(45) Date of publication and mention of the grant of the patent: **05.12.2018**
(21) Application number: 12776255.7
(22) Date of filing: 23.04.2012
(51) Int. Cl.: A61F 13/15, A61F 13/49, A61F 13/511, A61F 13/53, D04H 3/007, D04H 3/16, D01F 1/10, D01F 6/04

(54) **FIBER, NON-WOVEN FABRIC, AND USE THEREOF**
FASER, VLIESSTOFF UND VERWENDUNG DAVON
FIBRE, TISSU NON TISSÉ ET SON UTILISATION

(30) Priority: 27.04.2011 JP 2011099349
(43) Date of publication of application: 05.03.2014
(60) Divisional application: 18201491.0
(73) Proprietor: Mitsui Chemicals, Inc., Tokyo 105-7117 (JP)
(72) Inventor: TOMITA, Yoshihiko, Sodegaura-shi Chiba 299-0265 (JP); SUZUKI, Kenichi, Sodegaura-shi Chiba 299-0265 (JP); SUGAWARA, Daisuke, Sodegaura-shi Chiba 299-0265 (JP)
(74) Representative: Hoffmann Eitle
(86) International application number: PCT/JP2012/060867
(87) International publication number: WO 2012/147694

(56) References cited:
- EP-A2- 0 735 089
- WO-A1-95/33874
- WO-A1-2010/111491
- CN-A- 101 063 235
- JP-A- 2 053 950
- JP-A- 11 323 715
- JP-A- 2002 161 428
- JP-A- 2003 500 556
- JP-A- 2008 535 955
- JP-A- 2010 150 674
- US-A1- 2004 115 233
- US-A1- 2004 242 097
- US-A1- 2009 105 679

## Description

### TECHNICAL FIELD

The present invention relates to fibers which suppress the growth of harmful bacteria and reduce the occurrence of skin rashes and which are suitably used in products such as top sheets of hygiene materials such as disposable diapers and sanitary items, and to nonwoven fabrics including such fibers.

### BACKGROUND ART

Nonwoven fabrics obtained from olefin polymers, typically polypropylene, have excellent breathability, flexibility and lightweight properties and have been used widely in various applications including hygiene materials. Thus, the nonwoven fabrics require specific properties in accordance with the applications, and demands have been placed on the improvements of such properties.

For example, nonwoven fabrics used in hygiene materials such as disposable diapers require skin-friendly flexibility, quick urine absorption, breathability to release humidity, and antimicrobial properties to prevent skin rashes and foul odor due to bacteria and the like. In particular, skin rashes are very stressful to users. Thus, various remedies have been studied to suppress the growth of bacteria responsible for rashes as well as the occurrence of foul odor. In particular, it has been recently found that because skin is weakly acidic, rendering the skin environment mildly acidic promotes the growth of skin-friendly bacteria and can consequently suppress the growth of bacteria harmful to the skin, thereby reducing the occurrence of bacterial skin rashes.

For example, JP-A-2003-516778 (Patent Literature 1) discloses an approach in which a nonwoven fabric is impregnated with citric acid or sodium citrate as a pH control agent to maintain a pH environment similar to the pH of skin in contact with the nonwoven fabric. Because citric acid described in Patent Literature 1 is water soluble, however, the pH control agent such as citric acid is eluted and migrates toward an absorbent upon contact with urine. Thus, maintaining the pH is difficult. Further, for example, JP-A-2004-176225 (Patent Literature 2) discloses an approach which utilizes a deodorant fiber having a fiber treatment agent attached to the fiber, the fiber treatment agent including an amphoteric betaine compound, an adduct of a nonionic surfactant with an alkylene oxide, and/or an anionic surfactant such as a carboxylate salt. Because the fiber treatment agent in Patent Literature 2 is attached onto the fiber by application, a contact with urine results in the fiber treatment agent being partially eluted and migrating toward an absorbent. Thus, similarly to Patent Literature 1, this approach is not satisfactory in terms of the durability of effects.

Other various approaches have been proposed. For example, JP-A-2005-530857 (Patent Literature 3) proposes a method utilizing an antimicrobial composition comprising an antimicrobial active and an anionic surfactant, and JP-A-2002-505918 (Patent Literature 4) proposes an approach utilizing a skin care composition comprising a proton donating active component. However, any of these remedies have a problem in that effects are decreased with time. While a method utilizing an antimicrobial composition can reduce the bacterial growth temporarily by antimicrobial effects, the antimicrobial agents suppress not only the growth of harmful bacteria but also the growth of beneficial bacteria, resulting in the proliferation of harmful bacteria. Thus, the suppressive effects for diaper skin rashes in the art have been unsatisfactory and are to be improved.

WO2010/111491 A1 relates to compositions comprising a thermoplastic polyolefin polymer, an ionic surfactant and an anionic surfactant that can be processed into articles useful as wound contact materials or personal hygiene articles. The surfactants present in the composition are described as having the purpose of imparting wettability and activity against microorganisms.

EP 0 735 089 A2 discloses polyolefin compositions containing a polyolefin and at least one unsaturated compound in an amount that is effective in order to impart resistance against the deleterious effects of radiation. As said unsaturated compound, soy bean oil and safflower oil are mentioned. The compositions can be spun into yarns.

WO95/33874 A1 discloses degradable microfibers having a multilayer structure comprising at least one layer of polyolefin resin and at least one layer of polycaprolactone resin. As an optional component, a fatty acid can be present in the polymer composition.

CN 101063235 A discloses an antibacterial filamentary material comprising polypropylene and an antibacterial agent. The disclosed compositions exhibit activity against staphylococcus aureus and polybacteria. The antibacterial activity is imparted by means of an inorganic antiseptic powder comprising at least one component selected from ZnO, CuO and SnO.

US 2009/105679 A1 concerns a method for preparing thermoplastics to obtain a hydrophilic behaviour of the surface of objects made from said thermoplastics, as well as the uses of said thermoplastic objects with hydrophilized surfaces. It also concerns the additives used to obtain said surface hydrophily, said additives being mixed and jointly melted with the thermoplastics (mass-additivation process).

### CITATION LIST

### PATENT LITERATURE

Patent Literature 1: JP-A-2003-516778
Patent Literature 2: JP-A-2004-176225
Patent Literature 3: JP-A-2005-530857
Patent Literature 4: JP-A-2002-505918

### SUMMARY OF INVENTION

### TECHNICAL PROBLEM

The present inventors carried out studies in order to develop fibers which can suppress the growth of harmful bacteria and can continuously suppress the occurrence of skin rashes and which are suitably used in top sheets or second sheets of hygiene materials such as disposable diapers and sanitary items, as well as to develop nonwoven fabrics including such fibers. As a result, the present inventors have found that the above object can be accomplished with fibers obtained from an olefin polymer composition including an acid-form anionic surfactant, wherein the acid-form anionic surfactant is a phosphate ester surfactant in which a hydrophobic group has not less than 9 carbon atoms, and with nonwoven fabrics including such fibers.

### SOLUTION TO PROBLEM

The present invention provides a fiber which includes an olefin polymer composition including 100 parts by weight of an olefin polymer and 0.1 to 30 parts by weight of an acid-form anionic surfactant, wherein the acid-form anionic surfactant is a phosphate ester surfactant in which a hydrophobic group has not less than 9 carbon atoms, the acid-form anionic surfactant having been melt kneaded in the fiber.

### ADVANTAGEOUS EFFECTS OF INVENTION

Fibers of the invention and nonwoven fabrics including the fibers suppress the growth of harmful bacteria when used in hygiene materials such as diapers, and exhibit high durability of suppressive effects for skin rashes.

### DESCRIPTION OF EMBODIMENTS

### (Olefin polymers)

Olefin polymers which form the inventive fibers and nonwoven fabrics including the fibers are copolymers of propylene and α-olefins such as ethylene, 1-butene, 1-hexene, 4-methyl-1-pentene and 1-octene. Specific examples include propylene/ethylene random copolymers, propylene/ethylene/1-butene random copolymers (random polypropylenes) and propylene/1-butene random copolymers.

Propylene polymers are preferable because of excellent spinning stability during forming and good workability of nonwoven fabrics as well as because the obtainable nonwoven fabrics exhibit excellent breathability, flexibility, lightweight properties and heat resistance.

The olefin polymers in the invention may be blended with common additives or polymers other than the olefin polymers as required while still achieving the object of the invention. Exemplary additives include hydrophilicity enhancing or suppressing auxiliaries, antioxidants, weathering stabilizers, light stabilizers, antiblocking agents, lubricants, nucleating agents, pigments, softeners, water repellants, fillers and antimicrobial agents.

### 〈Propylene polymers〉

A preferred propylene polymer in the present invention is a copolymer of propylene and a small amount of one, or two or more kinds of α-olefins having 2 or more carbon atoms (except the C3 olefin), and preferably 2 to 8 carbon atoms (except the C3 olefin) such as ethylene, 1-butene, 1-pentene, 1-hexene, 1-octene and 4-methyl-1-pentene, and has a melting point (Tm) of not less than 125°C, and preferably in the range of 130 to 155°C.

The melt flow rate (MFR: ASTM D-1238, 230°C, 2160 g load) of the propylene polymer in the invention is not particularly limited as long as melt spinning is feasible. The melt flow rate, however, is usually in the range of 1 to 1000 g/10 min, preferably 5 to 500 g/10 min, and more preferably 10 to 100 g/10 min.

Of the propylene polymers in the invention, particularly preferable are propylene/α-olefin random copolymers of propylene and a small amount of one, or two or more kinds of α-olefins having 2 or more carbon atoms, and preferably 2 to 8 carbon atoms such as ethylene, 1-butene, 1-pentene, 1-hexene, 1-octene and 4-methyl-1-pentene, with a melting point (Tm) in the range of 125 to 155°C, and preferably 130 to 147°C. The use of such copolymers results in nonwoven fabrics exhibiting excellent flexibility and initial hydrophilicity.

In the propylene/α-olefin random copolymers, the content of α-olefins is not particularly limited as long as the melting point (Tm) is in the above range, but is usually in the range of 1 to 10 mol%, and more preferably 1 to 5 mol%.

### 〈Acid-form anionic surfactants〉

An acid-form anionic surfactant in the present invention is an anionic surfactant which is a phosphate ester surfactant in which a hydrophobic group has not less than 9 carbon atoms and which can act as an acid in the presence of water or moisture. In particular, an acid-form anionic surfactant having weak acidity is preferable as the acid-form anionic surfactant because it has a buffering capacity to reduce a change in pH. As long as the surfactants are of acid-form, incompletely neutralized salts, for example, partially neutralized salts such as monoalkyl phosphate ester salts may be used.

The acid-form anionic surfactants in the invention are phosphate ester surfactants in which a hydrophobic group has not less than 9 carbon atoms, preferably not less than 12 carbon atoms, more preferably not less than 13 carbon atoms, and particularly preferably 18 to 28 carbon atoms. Examples of the phosphate ester surfactants include alkyl phosphate esters which are esters formed between aliphatic alcohols and phosphoric acid; alkyl ether phosphate esters which are esters formed between adducts of aliphatic alcohols with alkylene oxides and phosphoric acid; and alkyl phenyl ether phosphate esters which are esters formed between adducts of aromatic alcohols with alkylene oxides and phosphoric acid.

A mixture of the acid-form anionic surfactants may be used in the invention. For example, acid-form phosphate ester surfactants with 18 to 28 carbon atoms may be alkyl phosphate esters which are esters formed between aliphatic alcohols having 18 to 28 carbon atoms and phosphoric acid; alkyl ether phosphate esters which are esters formed between adducts of aliphatic alcohols having 18 to 28 carbon atoms with alkylene oxides and phosphoric acid; alkyl phenyl ether phosphate esters which are esters formed between adducts of aromatic alcohols having 18 to 28 carbon atoms with alkylene oxides and phosphoric acid; or mixtures thereof. Acid-form phosphate ester surfactants with 9 or more carbon atoms, or mixtures thereof are preferable because better melt kneading properties can be obtained as well as because the acid-form anionic surfactants are suppressed from being eluted from nonwoven fabrics and the durability of effects is improved.

Specific examples of the acid-form phosphate ester surfactants in the invention include alkyl phosphate esters such as monostearyl phosphate ester, distearyl phosphate ester, monobehenyl phosphate ester, dibehenyl phosphate ester, monocetyl phosphate ester, dicetyl phosphate ester, monolauryl phosphate ester, dilauryl phosphate ester, monooctyl phosphate ester and dioctyl phosphate ester; alkyl ether phosphate esters such as mono (polyoxyethylene stearyl ether) phosphate ester, di(polyoxyethylene stearyl ether) phosphate ester, mono(polyoxyethylene lauryl ether) phosphate ester and di(polyoxyethylene lauryl ether) phosphate ester; and alkylphenyl ether phosphate esters such as mono(polyoxyethylene stearylphenyl ether) phosphate ester, di(polyoxyethylene stearylphenyl ether) phosphate ester, mono(polyoxyethylene laurylphenyl ether) phosphate ester and di(polyoxyethylene laurylphenyl ether) phosphate ester.

### (Nonionic surfactants)

Various known nonionic surfactants may be used as the nonionic surfactants in the invention without limitation as long as the addition of such nonionic surfactants results in fibers exhibiting hydrophilicity on the surface. Specific examples of the nonionic surfactants include fatty acid glycerides, alkoxylated alkylphenols, polyoxyalkylene fatty acid esters, alkyl polyoxyethylene alcohols, fatty acid amides, polyoxyalkylene sorbitan fatty acid esters, sucrose fatty acid esters, sorbitan fatty acid esters, fatty acid alkanolamides, polyoxyalkylene alkyl ethers, polyoxyalkylene alkylphenyl ethers, alkyl polyglucosides, fatty acid diethanolamides and alkyl monoglyceryl ethers.

### 〈Olefin polymer compositions〉

An olefin polymer composition in the invention includes 100 parts by weight of the olefin polymer and 0.1 to 30 parts by weight, preferably 1 to 15 parts by weight, and more preferably 1.5 to 10 parts by weight of the acid-form anionic surfactant.

If the amount of the acid-form anionic surfactant is less than 0.1 part by weight, the environment around the fibers will not be adequately rendered weakly acidic. As a result, nonwoven fabrics including such fibers may fail to reduce the occurrence of rashes when used in hygiene materials such as sanitary items and diapers. The upper limit of the amount is not particularly limited. However, any amount exceeding 30 parts by weight results in the saturation of the effects of rendering the environment around the fibers weakly acidic as well as in an increase in the amount of the acid-form anionic surfactant exuding to the surface of the obtainable fibers and nonwoven fabrics including the fibers, thus deteriorating forming processability.

In addition to the acid-form anionic surfactant, the olefin polymer composition of the invention may contain the nonionic surfactant in an amount of 0.1 to 30 parts by weight, and preferably 0.5 to 15 parts by weight with respect to 100 parts by weight of the olefin polymer. This configuration can improve the hydrophilicity of the obtainable fibers and can prevent a rapid change in pH due to the acid-form anionic surfactant.

In the preparation of the inventive olefin polymer composition, masterbatch pellets may be formed which contain the acid-form anionic surfactant at a high concentration exceeding 5 wt%, for example, 10 to 40 wt%. This masterbatching advantageously facilitates the addition of the acid-form anionic surfactant and the mixing thereof with the olefin polymer in the production of fibers and nonwoven fabrics. An olefin polymer mixed with the acid-form anionic surfactant to give the masterbatch may be any of the olefin polymers described above or any of other olefin polymers. The MFR of the olefin polymer used in the masterbatch may be selected appropriately in accordance with the target fibers and nonwoven fabrics including the fibers.

The inventive olefin polymer composition may contain usual additives or polymers other than the olefin polymers as required while still achieving the object of the invention. Exemplary additives include antioxidants, weathering stabilizers, light stabilizers, antiblocking agents, lubricants, nucleating agents and pigments.

### 〈Fibers and nonwoven fabrics〉

Fibers of the invention and nonwoven fabrics including the fibers are formed of the olefin polymer composition containing the olefin polymer, the acid-form anionic surfactant, and preferably the nonionic surfactant.

In particular, fibers in which the acid-form anionic surfactant has been melt kneaded in the olefin polymer advantageously exhibit prolonged effects of reducing the occurrence of skin rashes. When the nonionic surfactant is used, this surfactant may be melt kneaded together with the acid-form anionic surfactant. This configuration improves melt kneading properties and allows the nonionic surfactant and the acid-form anionic surfactant to be dispersed uniformly on the surface of fibers.

The inventive fibers usually have a fineness in the range of 0.5 to 5 d. The inventive fibers may be discontinuous fibers, or may be preferably continuous fibers due to advantages such as no fiber detachment from the nonwoven fabrics.

The inventive fibers may have a circular cross section or a modified cross section such as a V-shaped, X-shaped or T-shaped cross section.

The fibers of the invention may be side-by-side crimped fibers which are formed of olefin polymer compositions including at least two or more components. Preferred configurations include side-by-side crimped fibers formed of a propylene homopolymer and a propylene/ethylene random copolymer as the olefin polymers.

Alternatively, the fibers of the invention may be concentric or eccentric fibers. In such cases, the acid-form anionic surfactant and preferably the nonionic surfactant may be added to both or one of the core and the sheath.

Still alternatively, the inventive fibers may be mixed with other fibers to form mixed fiber nonwoven fabrics. For example, the inventive fibers such as the above crimped fibers may be mixed with other uncrimped fibers to form mixed fiber nonwoven fabrics.

The nonwoven fabrics of the invention usually have a basis weight in the range of 10 to 30 g/m², and preferably 15 to 25 g/m².

Depending on applications, the nonwoven fabrics of the invention may be entangled by various known entangling methods, for example, by needle punching, water jetting or ultrasonicating or by partial thermal fusion bonding through hot embossing with an embossing roll or by blowing of hot air through the fibers. These entangling methods may be used singly, or a plurality of these entangling methods may be used in combination.

When the nonwoven fabrics are thermally fusion bonded by hot embossing, the emboss area percentage is usually in the range of 3 to 30%, and preferably 5 to 20%. Exemplary shapes of the bosses include round shapes, elliptical shapes, oval shapes, square shapes, rhombic shapes, rectangular shapes, quadrangular shapes, quilt shapes, grid shapes, tortoiseshell shapes, and continuous shapes based on these shapes.

Depending on various applications, the nonwoven fabrics of the invention may be used singly or as laminates (stacks) in combination with other layers.

Specific examples of the additional layers laminated with the inventive nonwoven fabrics include knitted fabrics, woven fabrics, nonwoven fabrics, films and paper products. Such additional layers may be laminated (bonded) to the nonwoven fabrics of the invention by any of various known methods, for example, thermal fusion bonding methods such as hot embossing and ultrasonic fusion bonding, mechanical entangling methods such as needle punching and water jetting, bonding methods with adhesives such as hot melt adhesives and urethane adhesives, and extrusion lamination. The nonwoven fabrics of the invention maintain rash suppressive effects even after subjected to steps bringing the nonwoven fabrics into contact with high-temperature substances such as hot melt adhesives or during extrusion lamination. Thus, high convenience can be obtained in controlling product quality.

The nonwoven fabrics laminated to the nonwoven fabrics of the invention may be any of various known nonwoven fabrics such as spunbonded nonwoven fabrics, meltblown nonwoven fabrics, wet nonwoven fabrics, dry nonwoven fabrics, dry pulp nonwoven fabrics, flash-spun nonwoven fabrics and split-fiber nonwoven fabrics.

Exemplary materials for such nonwoven fabrics include various known thermoplastic resins, for example, polyolefins which are homopolymers and copolymers of α-olefins such as ethylene, propylene, 1-butene, 1-hexene, 4-methyl-1-pentene and 1-octene, with specific examples including high-pressure low-density polyethylenes, linear low-density polyethylenes (LLDPE), high-density polyethylenes, polypropylenes, polypropylene random copolymers, poly(1-butene), poly(4-methyl-1-pentene), ethylene/propylene random copolymers, ethylene/1-butene random copolymers and propylene/1-butene random copolymers, polyesters (such as polyethylene terephthalates, polybutylene terephthalates and polyethylene naphthalates), polyamides (such as nylon-6, nylon-66 and poly(meta-xylene adipamide), polyvinyl chlorides, polyimides, ethylene/vinyl acetate copolymers, polyacrylonitriles, polycarbonates, polystyrenes, ionomers, thermoplastic polyurethanes, and mixtures of these resins. Of these, preferred resins include high-pressure low-density polyethylenes, linear low-density polyethylenes (LLDPE), high-density polyethylenes, polypropylenes, polypropylene random copolymers, polyethylene terephthalates and polyamides.

When spunbonded nonwoven fabrics of crimped fibers are laminated to the inventive nonwoven fabrics, the obtainable laminates exhibit excellent flexibility, bulkiness and touch. When stretched nonwoven fabrics composed of mixed fibers of continuous thermoplastic resin fibers and continuous thermoplastic elastomer fibers are laminated to the inventive nonwoven fabrics, the obtainable laminates exhibit excellent stretchability in addition to the above properties.

When the inventive nonwoven fabrics are used in top sheets, second sheets or absorbent-wrapping sheets (core wraps) in absorptive articles, for example, disposable diapers, it is preferable that the fineness be in the range of 0.5 to 3 d and the basis weight be in the range of 7 to 50 g/m².

In the case where the inventive nonwoven fabrics are used in absorbent-wrapping sheets (core wraps) in absorptive articles, the inventive nonwoven fabrics may be used as laminates in combination with the above-described meltblown nonwoven fabrics.

The films laminated to the nonwoven fabrics of the invention are preferably breathable (moisture permeable) films in order to better take advantage of rash suppressive effects that are characteristics of the inventive nonwoven fabrics. Various known breathable films may be used, with examples including moisture permeable films of thermoplastic elastomers such as polyurethane elastomers, polyester elastomers and polyamide elastomers; and porous films obtained by drawing thermoplastic resin films containing inorganic or organic fine particles to create pores in the films. Preferred thermoplastic resins used for the porous films are polyolefins such as high-pressure low-density polyethylenes, linear low-density polyethylenes (LLDPE), high-density polyethylenes, polypropylenes, polypropylene random copolymers, and compositions of these resins.

The inventive nonwoven fabrics may be subjected to secondary processing such as gear processing, printing, coating, lamination, heat treatment or shaping processing while still achieving the object of the invention.

### 〈Processes for producing nonwoven fabrics〉

In the case where discontinuous fibers are used as materials for the nonwoven fabrics, the nonwoven fabrics may be produced by any of various known processes such as wet processes and dry processes (carding).

### 〈Processes for producing continuous fiber nonwoven fabrics〉

Continuous fiber nonwoven fabrics according to the present invention may be produced by any of various known nonwoven fabric producing processes. A spunbonding process is preferable because of excellent productivity.

In the case of a spunbonding process, an inventive continuous fiber nonwoven fabric including monofilaments of the olefin polymer composition may be produced in the following manner. The composition is molten in a single extruder at 180 to 250°C, preferably 190 to 230°C, and is extruded through a spinneret with spinning nozzles that has been set at 180 to 250°C, preferably 190 to 230°C, at a throughput per orifice of 0.4 to 1.5 g/min, preferably 0.5 to 0.8 g/min, thereby spinning fibers . The fibers that have been spun are cooled with cooling air at 10 to 40°C, preferably 20 to 40°C, and are attenuated to a desired fineness with high velocity air at 2000 to 7000 m/min, preferably 3000 to 6000 m/min. The fibers are then deposited onto a collection belt to a desired thickness (basis weight) and are entangled by an entangling method such as needle punching, water jetting or ultrasonicating, or by partial thermal fusion bonding through hot embossing with an embossing roll or by blowing of hot air through the fibers.

### 〈Absorptive articles〉

Absorptive articles according to the invention utilize the aforementioned nonwoven fabrics, preferably continuous fiber nonwoven fabrics, in top sheets and/or second sheets, or absorbent-wrapping sheets (core wraps). Examples of the inventive absorptive articles include disposable diapers, underwear or sanitary items, urine collection pads, and pet sheets.

In particular, when the nonwoven fabrics including fibers of the olefin polymer composition are used in top sheets, second sheets or absorbent-wrapping sheets (core wraps) in disposable diapers, the olefin polymer composition preferably includes 0.5 to 30 parts by weight of the acid-form anionic surfactant, or 0. 5 to 30 parts by weight of the acid-form anionic surfactant plus 0.5 to 30 parts by weight of the nonionic surfactant, with respect to 100 parts by weight of the olefin polymer.

### EXAMPLES

The present invention will be described in greater detail based on examples hereinbelow without limiting the scope of the invention to such examples.

The degree of rash suppressive effects in examples and comparative examples was evaluated by the following method.

### [Rash evaluation method]

The extent of rashes was evaluated in the following manner.

Stool sampled from a healthy three year old child (male) was diluted to 1% with distilled water and was centrifuged at 3000 rpm for 15 minutes using cooling high-speed centrifuge H-201FR manufactured by KOKUSAN Co., Ltd. The supernatant liquid was collected as an evaluation liquid. To the evaluation liquid, a nonwoven fabric sample produced in any of the following examples and comparative examples was soaked for 1 minute or 30 minutes and was thereafter attached to the skin of three rabbits provided in accordance with ISO 10993-10 (Tests for irritation and delayed-type hypersensitivity). The skin condition after 48 hours was observed.

### [Evaluation criteria]

1 point: Substantially no changes were observed on the skin of all the three rabbits.
2 points: Slight edema and spots were found on one or two rabbits.
3 points: Slight edema and spots were found on all the three rabbits.
4 points: Edema and spots were found on one or two rabbits.
5 points: Edema and spots were found on all the three rabbits.
6 points: Marked edema and spots were found on all the three rabbits.

### [Extrudability]

1 point: The composition was excellently engaged with an extruder screw.
2 points: The composition was engaged with an extruder screw with difficulties. Variations in nozzle pressure occurred.

### [Example 1] (not according to the invention)

A composition was prepared as described below which contained 100 parts by weight of an olefin polymer and an amount described in Table 1 of an acid-form anionic surfactant or a nonionic surfactant. 10 Parts by weight of stearic acid (product name: LUNAC S-90V manufactured by Kao Corporation) as an acid-form anionic surfactant, and 10 parts by weight of an adduct of stearyl alcohol with ethylene oxide (IRGASURF HL560 manufactured by BASF (former Ciba)) as a nonionic surfactant were melt kneaded and extruded at 230°C together with 79.95 parts by weight of a propylene/ethylene random copolymer (PP-1) having a melting point (Tm) of 142°C and a MFR of 60 g/10 min and 0.05 parts by weight of an antioxidant (product name: IRGAFOS 168 manufactured by BASF (former Ciba)). Thus, pellets of a masterbatch (MB-1) were prepared.

Next, 40 parts by weight of the MB-1 was admixed with 60 parts by weight of the PP-1 to give a propylene polymer composition (a composition 1).

In order to evaluate extrusion performance, the composition 1 was placed into a single screw extruder (screw diameter 30 mm, L/D = 30) and was molten by heating with a throughput of 4.4 kg/hr at a resin temperature of 200°C. The composition 1 was engaged with the screw of the extruder as excellently as when the surfactants were not added.

Next, the composition 1 was melt spun by a spunbonding process to form a continuous fiber nonwoven fabric. After the spinning, the nonwoven fabric was subjected to hot embossing using an embossing roll with rhombic bosses having an emboss area percentage of 18% and an emboss area (an area per boss) of 0.41 mm², at an embossing roll temperature and a flat roll temperature of 125°C and a linear pressure of 60 N/mm. Thus, a continuous fiber nonwoven fabric with a basis weight of 20 g/m² was obtained. The obtained continuous fiber nonwoven fabric was tested by the above rash evaluation method to evaluate the extent of rashes. The results are described in Table 1.

### [Examples 2 to 6] (examples 2, 3, 6 are not according to the invention)

Continuous fiber nonwoven fabrics were obtained in the same manner as in Example 1, except that the following compounds were used as the acid-form anionic surfactant and the nonionic surfactant, as well as that the amounts were changed as described in Table 1. The obtained continuous fiber nonwoven fabrics were tested by the above rash evaluation method to evaluate the extent of rashes. The results are described in Table 1.

Myristic acid (C14): LUNAC MY-98 (product name) manufactured by Kao Corporation
Oleth-4 phosphate (C18): PHOSPHANOL RB-410 (product name) manufactured by TOHO Chemical Industry Co., Ltd.

### [Example 7]

A composition was prepared as described below which contained 100 parts by weight of an olefin polymer and an amount described in Table 1 of an acid-form anionic surfactant or a nonionic surfactant. 5 Parts by weight of dodecyl phosphoric acid (manufactured by TAKEMOTO OIL & FAT CO., LTD.) as an acid-form anionic surfactant, and 14 parts by weight of poly(oxyethylene) 1,6-hexanediol dilaurate (manufactured by TAKEMOTO OIL & FAT CO., LTD.) and 1 part by weight of poly(oxyethylene) sorbitan trioleate ester (manufactured by TAKEMOTO OIL & FAT CO., LTD.) as nonionic surfactants were melt kneaded and extruded at 180°C together with 79.95 parts by weight of a propylene/ethylene random copolymer (PP-1) having a melting point (Tm) of 142°C and a MFR of 60 g/10 min and 0.05 parts by weight of an antioxidant (product name: IRGANOX 1010 manufactured by BASF (former Ciba)). Thus, pellets of a masterbatch (MB-2) were prepared.

Next, 20 parts by weight of the MB-2 was admixed with 80 parts by weight of the PP-1 to give a propylene polymer composition (a composition 2).

In order to evaluate extrusion performance, the composition 2 was placed into a single screw extruder (screw diameter 30 mm, L/D = 30) and was molten by heating with a throughput of 4.4 kg/hr at a resin temperature of 200°C. The composition 2 was engaged with the screw of the extruder as excellently as when the surfactants were not added.

Next, the composition 2 was melt spun by a spunbonding process to form a continuous fiber nonwoven fabric. After the spinning, the nonwoven fabric was subjected to hot embossing using an embossing roll with rhombic bosses having an emboss area percentage of 18% and an emboss area (an area per boss) of 0.41 mm², at an embossing roll temperature and a flat roll temperature of 125°C and a linear pressure of 60 N/mm. Thus, a continuous fiber nonwoven fabric with a basis weight of 20 g/m² was obtained. The obtained continuous fiber nonwoven fabric was tested by the above rash evaluation method to evaluate the extent of rashes. The results are described in Table 1.

### [examples 8 and 9] (example 8 is not according to the invention)

Continuous fiber nonwoven fabrics were obtained in the same manner as in Example 7, except that the following compounds were used as the acid-form anionic surfactants while the nonionic surfactants used in Example 7 were not changed, as well as that the amounts were changed as described in Table 1. The obtained continuous fiber nonwoven fabrics were tested by the above rash evaluation method to evaluate the extent of rashes. The results are described in Table 1.

### Anionic surfactants

Octyl phosphoric acid (C8) : manufactured by TAKEMOTO OIL & FAT CO., LTD.

Octadecan-1-yl dihydrogen phosphate (C18) : manufactured by TAKEMOTO OIL & FAT CO., LTD.

Dioctadecyl hydrogen phosphate (C18): manufactured by TAKEMOTO OIL & FAT CO., LTD.

### [Table 1]

**Table 1**

| Ex. | Process | Acid-form anionic surfactants | | | Nonionic surfactants | | Rash evaluation | | Extrudability |
|---|---|---|---|---|---|---|---|---|---|
| | | Types | Number of carbon atoms in hydrophobic group | Amt. (wt ₊ pts.) | Types | Amt. (wt. pts.) | 1 Min soak | 30 Min soak | |
| *1 | Kneading | Stearic acid | C18 | 4 | EO adduct of stearyl alcohol | 4 | 2 | 2 | 1 |
| *2 | Kneading | Stearic acid | C18 | 4 | | | 2 | 2 | 1 |
| *3 | Kneading | Myristic acid | C14 | 4 | | | 2 | 3 | 1 |
| 4 | Kneading | oleth-4 phosphate | C18 | 4 | EO adduct of stearyl alcohol | 4 | 1 | 1 | 1 |
| 5 | Kneading | Oleth-4 phosphate | C18 | 4 | | | 1 | 2 | 1 |
| *6 | Kneading | Stearic acid | C18 | 2 | EO adduct of stearyl alcohol | 4 | 3 | 3 | 1 |
| 7 | Kneading | Dodecyl phosphoric acid | C12 | 1 | Poly(oxyethylene) 1,6-hexanediol dilaurate | 3 | 1 | 1 | 1 |
| | | | | | Poly(oxyethylene) sorbitan trioleate ester | 0.2 | | | |
| *8 | Kneading | Octyl phosphoric acid | C8 | 1 | Poly(oxyethylene) 1,6-hexanediol dilaurate | 3 | 1 | 2 | 2 |
| | | | | | Poly(oxyethylene) sorbitan trioleate ester | 0.2 | | | |
| 9 | Kneading | Octadecan-1-yl dihydrogen phosphate | C18 | 1 | Poly(oxyethylene) 1,6-hexanediol dilaurate | 3 | 1 | 1 | 1 |
| | | Dioctadecyl hydrogen phosphate | C16 | | Poly(oxyethylene) sorbitan trioleate ester | 0.2 | | | |

| | | | | | | | | | |
|---|---|---|---|---|---|---|---|---|---|
| * : not according to the invention | | | | | | | | | |

### [Comparative Example 1]

A continuous fiber nonwoven fabric was obtained in the same manner as in Example 1, except that the nonionic surfactant used in Example 1 alone was added and any acid-form anionic surfactants were not added. The obtained continuous fiber nonwoven fabric was tested by the above rash evaluation method to evaluate the extent of rashes. The results are described in Table 2.

### [Comparative Examples 2 to 6]

The PP-1 was melt spun by a spunbonding process to form a continuous fiber nonwoven fabric. After the spinning, the nonwoven fabric was subjected to hot embossing using an embossing roll with rhombic bosses having an emboss area percentage of 18% and an emboss area (an area per boss) of 0.41 mm², at an embossing roll temperature and a flat roll temperature of 125°C and a linear pressure of 60 N/mm. Thus, continuous fiber nonwoven fabrics (NW-1) with a basis weight of 20 g/m² were obtained. In Comparative Examples 2 to 6 described in Table 2, the following compounds were dissolved in an aqueous isopropyl alcohol solution with a concentration adjusted such that the compounds would exhibit solubility in the solution, thereby preparing coating liquids. The coating liquids were each applied to the nonwoven fabrics NW-1 and were thereafter dried. By repeating the application and drying, the dry weights were controlled to be the prescribed amounts of the compounds added which are described in Table 2. The obtained nonwoven fabrics were tested by the above rash evaluation method to evaluate the extent of rashes. The results are described in Table 2.

### Anionic surfactants

Citric acid (C6): reagent manufactured by Wako Pure Chemical Industries, Ltd.
Octylic acid (C8) : reagent manufactured by KH Neochem Co., Ltd.
Stearyl alcohol (C18): KALCOL 8098 (product name) manufactured by Kao Corporation
Dodecyl phosphoric acid (C12): manufactured by TAKEMOTO OIL & FAT CO., LTD.

### Nonionic surfactants

Poly(oxyethylene) 1,6-hexanediol dilaurate: manufactured by TAKEMOTO OIL & FAT CO., LTD.
Poly(oxyethylene) sorbitan trioleate ester (manufactured by TAKEMOTO OIL & FAT CO., LTD.)

### [Table 2]

**Table 2**

| Comp. Ex. | Process | Acid-form anionic surfactants | | | Nonionic surfactants | | Rash evaluation | |
|---|---|---|---|---|---|---|---|---|
| | | Types | Number of carbon atoms | Amt. (wt. pts.) | Types | Amt. (wt. pts.) | 1 Min soak | 30 Min soak |
| 1 | Kneading | | | | EO adduct of stearyl alcohol | 4 | 6 | 6 |
| 2 | Application | Na stearate | C18 | 4 | EO adduct of stearyl alcohol | 4 | 6 | 6 |
| 3 | Application | Citric acid | C6 | 4 | | | 4 | 6 |
| 4 | Application | Octylic acid | C8 | 4 | | | 5 | 6 |
| 5 | Application | Stearyl alcohol | C18 | 4 | | | 6 | 6 |
| 6 | Application | Dodecyl phosphoric acid | C12 | 1 | Poly(oxyethylene) 1,6-hexanediol dilaurate | 3 | 1 | 4 |
| | | | | | Poly(oxyethylene) sorbitan trioleate ester | 0.2 | | |

### INDUSTRIAL APPLICABILITY

The fibers of the present invention and the nonwoven fabrics including the fibers can be suitably used as top sheets or second sheets of hygiene materials such as disposable diapers and sanitary items.

## Claims

1. A fiber comprising an olefin polymer composition comprising 100 parts by weight of an olefin polymer and 0.1 to 30 parts by weight of an acid-form anionic surfactant, the acid-form anionic surfactant having been melt kneaded in the fiber,
wherein the acid-form anionic surfactant is a phosphate ester surfactant in which a hydrophobic group has not less than 9 carbon atoms, and
the olefin polymer is a propylene/α-olefin random copolymer comprising propylene and an α-olefin and having a melting point (Tm) in the range of 125 to 155°C.

2. The fiber according to claim 1, wherein the olefin polymer composition comprises 0.1 to 30 parts by weight of a nonionic surfactant.

3. The fiber according to claim 1 or claim 2, wherein the fiber is a continuous fiber.

4. A nonwoven fabric comprising the fiber described in claim 3.

5. An absorptive article in which the nonwoven fabric described in claim 4 is used in a top sheet and/or a second sheet.

6. An absorptive article in which the nonwoven fabric described in claim 4 is used in an absorbent-wrapping sheet.

## Patentansprüche

1. Faser, umfassend eine Olefinpolymerzusammensetzung, umfassend 100 Gewichtsteile eines Olefinpolymers und 0,1 bis 30 Gewichtsteile eines säureförmigen anionischen Tensids, wobei das säureförmige anionische Tensid in der Faser schmelzgeknetet wurde,
wobei das säurefömige anionische Tensid ein Phosphatester-Tensid ist, in dem eine hydrophobe Gruppe nicht weniger als 9 Kohlenstoffatome aufweist, und
das Olefinpolymer ein statistisches Propylen/α-Olefin-Copolymer, umfassend Propylen und ein α-Olefin, ist und einen Schmelzpunkt (Tm) im Bereich von 125 bis 155°C aufweist.

2. Faser gemäß Anspruch 1, wobei die Olefinpolymerzusammensetzung 0,1 bis 30 Gewichtsteile eines nichtionischen Tensids umfasst.

3. Faser gemäß Anspruch 1 oder Anspruch 2, wobei die Faser eine kontinuierliche Faser ist.

4. Vliesstoff, umfassend die in Anspruch 3 beschriebene Faser.

5. Absorbierender Artikel, bei dem der in Anspruch 4 beschriebene Vliesstoff in einer Oberschicht und/oder in einer zweiten Schicht verwendet wird.

6. Absorbierender Artikel, bei dem der in Anspruch 4 beschriebene Vliesstoff in einem Sheet zum Einhüllen des Absorbers verwendet wird.

## Revendications

1. Fibre comprenant une composition de polymère oléfinique comprenant 100 parties en poids d'un polymère oléfinique et 0,1 à 30 parties en poids d'un tensioactif anionique de forme acide, le tensioactif anionique de forme acide ayant été malaxé à l'état fondu dans la fibre,
dans laquelle le tensioactif anionique de forme acide est un tensioactif d'ester phosphorique dans lequel un groupe hydrophobe n'a pas moins de 9 atomes de carbone, et
le polymère oléfinique est un copolymère aléatoire de propylène/α-oléfine comprenant du propylène et une α-oléfine et ayant un point de fusion (Tm) dans la plage de 125 à 155 °C.

2. Fibre selon la revendication 1, dans laquelle la composition de polymère oléfinique comprend 0,1 à 30 parties en poids d'un tensioactif non ionique.

3. Fibre selon la revendication 1 ou la revendication 2, dans laquelle la fibre est une fibre continue.

4. Tissu non tissé comprenant la fibre décrite dans la revendication 3.

5. Article absorbant dans lequel le tissu non tissé décrit dans la revendication 4 est utilisé dans une feuille supérieure et/ou une seconde feuille.

6. Article absorbant dans lequel le tissu non tissé décrit dans la revendication 4 est utilisé dans une feuille d'emballage absorbant.
